# EUROPEAN PATENT SPECIFICATION

(11) **EP 3 797 422 B1**
(45) Date of publication and mention of the grant of the patent: **31.07.2024**
(21) Application number: 19739381.2
(22) Date of filing: 22.05.2019
(51) Int. Cl.: G16H 40/63, G16H 20/40, G16H 30/40

(54) **SYSTEM AND METHOD OF UTILIZING SURGICAL TOOLING EQUIPMENT WITH GRAPHICAL USER INTERFACES**
SYSTEM UND VERFAHREN ZUR VERWENDUNG VON CHIRURGISCHEN WERKZEUGEN MIT GRAFISCHEN BENUTZEROBERFLÄCHEN
SYSTEME ET PROCEDE D'UTILISATION D'UN OUTIL CHIRURGICAL AVEC DES INTERFACES GRAPHIQUES

(30) Priority: 23.05.2018 US 201862675584 P
(43) Date of publication of application: 31.03.2021
(73) Proprietor: Alcon Inc., 1701 Fribourg (CH)
(72) Inventor: ZIEGER, Peter, 14513 Teltow (DE); GRUENDIG, Martin, 14513 Teltow (DE)
(74) Representative: Mooser, Sebastian Thomas
(86) International application number: PCT/IB2019/054242
(87) International publication number: WO 2019/224746

(56) References cited:
- US-A1- 2017 196 453
- OSHIRO YUKIO ET AL: "Novel imaging using a touchless display for computer-assisted hepato-biliary surgery", SURGERY TODAY, SPRINGER, JP, vol. 47, no. 12, 20 May 2017 (2017-05-20), pages 1512 - 1518, XP036350521, ISSN: 0941-1291, [retrieved on 20170520], DOI: 10.1007/S00595-017-1541-7

## Description

### BACKGROUND

### Field of the Disclosure

This disclosure relates to quality assurance in medical procedures and more particularly to systems and methods for authenticating patient information with the medical procedure or process elements of the medical procedure.

### Description of the Related Art

Computer systems can assist surgeons in surgeries. The computer systems provide graphical user interfaces. However, in a sterile environment surgeons cannot easily touch non-sterile devices, such as interfaces to computer systems. Currently, surgeons have different possibilities to interact with interfaces to computer systems, such as foot pedals, a surgical assistant (e.g., medical personnel), and one-time disposals (e.g. Q-tips) to interact on a touch-screen and/or a keyboard. These solutions can be error prone and can lead to a wrong input. For example, during the interaction with a computer system, a surgeon may have to physically move his or her hand and/or head from a patient to a computer system interface to ensure that his or her computer system input is correct. This can be a potential distraction during a surgery, which can lead to unforeseen and/or negative surgical results.

Document US 2017/196453 A1 discloses an ophthalmic surgical system having a surgical microscope operable to provide a field of view of a surgical site to a user, and a display device in communication with the surgical microscope, the display device being operable to output a graphical overlay into the field of view of the surgical microscope. The system further includes a tool operable to perform a surgical task associated with the surgical site while the user visualizes the field of view using the surgical microscope, wherein the graphical overlay includes an adjustment field operable to modify one or more configurable parameters associated with the tool.

In document Oshiro Yukio et al, "Novel imaging using a touchless display for computer-assisted hepato-biliary surgery", Surgery Today, SPRINGER, JP, vol. 47, no. 12, 20 May 2017, pages 1512-1518, a touchless display system is described that allows the user to control medical imaging software via hand gestures in the air, wherein patient's CT data is generally observed on a display during a surgery.

### SUMMARY

The present invention concerns a system according to claim 1, at least one non-transitory computer readable storage medium according to claim 6, and a method according to claim 11. In particular, the disclosure provides a system able to display a graphical user interface via a display and able to receive first user input that indicates that surgical tooling equipment is to be utilized as a pointer associated with the graphical user interface. For example, the surgical tooling equipment to be utilized as the pointer may include a scalpel, a Q-tip, tweezers, etc. The system may include or may be coupled to the display that displays the graphical user interface. The system may include a microscope integrated display that includes the display, which displays the graphical user interface. The system may further receive first multiple images from at least one image sensor and may further determine, from the first multiple images, a digital model of the surgical tooling equipment. For example, the digital model may include a pattern of the surgical tooling equipment. In determining the digital model, the digital model may be trained based at least on the first multiple images. For example, training the digital model may include determining data associated with the surgical tooling equipment in a portion of each of the first multiple images. The surgical tooling equipment may be moved to a registration area displayed by the graphical user interface. User input may be received that indicates that the surgical tooling equipment is present in the registration area. For example, image data associated with the registration area of the graphical user interface may be utilized as or with training data for the digital model of the surgical tooling equipment.

The system may further receive user input that selects an icon of the graphical user interface. In one example, the user input that selects the icon may include an actuation of a foot pedal. In another example, the user input that selects the icon may include a movement of the surgical tooling equipment. The system may further receive second multiple images via the at least one image sensor, which may be utilized in determining a pattern of movement of the surgical tooling equipment that is utilizable to select of the icon of the graphical user interface.

The present disclosure may further include a non-transient computer-readable memory device with instructions that, when executed by a processor of a system, cause the system to perform the above steps. The present disclosure further includes a system or a non-transient computer-readable memory device as described above with one or more of the following additional features, which may be used in combination with one another unless clearly mutually exclusive: i) as the processor executes the instructions, the system may be further able to display a graphical user interface via a display; ii) as the processor executes the instructions, the system may be further able to receive first user input that indicates that surgical tooling equipment is to be utilized as a pointer associated with the graphical user interface; iii) as the processor executes the instructions, the system may be further able to receive first multiple images from at least one image sensor; iv) as the processor executes the instructions, the system may be further able to determine a digital model of the surgical tooling equipment, from the first multiple images, that includes a pattern of the surgical tooling equipment; v) when the system determines the digital model of the surgical tooling equipment, the system may be further able to train the digital model based at least on the first multiple images; vi) when the system trains the digital model, the system may be further able to determine data associated with the surgical tooling equipment in a portion of each of the first multiple images; and vii) when the system displays the graphical user interface via the display, the system may be further able to indicate an area, via the graphical user interface, associated with each portion of each of the first multiple images.

Any of the above systems may be able to perform any of the above methods and any of the above non-transient computer-readable memory devices may be able to cause a system to perform any of the above methods. Any of the above methods may be implemented on any of the above systems or using any of the above non-transient computer-readable memory devices.

### BRIEF DESCRIPTION OF THE DRAWINGS

For a more complete understanding of the present disclosure and its features and advantages, reference is now made to the following description, taken in conjunction with the accompanying drawings, which are not drawn to scale, and in which:
FIG. 1A illustrates an example of a system;
FIG. 1B illustrates an example of a microscope integrated display and examples of surgical tooling equipment;
FIG. 2 illustrates an example of a computer system;
FIGs. 3A and 3B illustrate examples of a graphical user interface;
FIGs. 4A and 4B illustrate examples of registration areas;
FIG. 4C illustrates an example registering a movement pattern;
FIG. 4D illustrates an example receiving a movement pattern;
FIG. 4E illustrates another example receiving a movement pattern;
FIG. 5 illustrates an example of a method utilizing surgical tooling equipment with a graphical user interface; and
FIG. 6 illustrates another example of a method utilizing surgical tooling equipment with a graphical user interface.

### DETAILED DESCRIPTION

In the following description, details are set forth by way of example to facilitate discussion of the disclosed subject matter. It should be apparent to a person of ordinary skill in the field, however, that the disclosed embodiments are examples and not exhaustive of all possible embodiments.

As used herein, a reference numeral refers to a class or type of entity, and any letter following such reference numeral refers to a specific instance of a particular entity of that class or type. Thus, for example, a hypothetical entity referenced by ` 12A' may refer to a particular instance of a particular class/type, and the reference '12' may refer to a collection of instances belonging to that particular class/type or any one instance of that class/type in general.

A surgeon may be in a sterile surgical environment. The surgeon may use his or her surgical tooling equipment to control and/or direct a graphical user interface (GUI). The GUI may be utilized to control a workflow associated with a surgery. In utilizing surgical tooling equipment to control and/or direct a GUI, a device may determine one or more shapes of surgical tooling equipment. For example, one or more cameras may provide one or more images to the device. The device may determine the surgical tooling equipment from the one or more images from the one or more cameras. The device may track one or more movements of the surgical tooling equipment. For example, the device may track one or more movements of the surgical tooling equipment based at least on the one or more images from the one or more cameras.

The one or more movements of the surgical tooling equipment that are tracked may be utilized in interacting with a GUI. In one example, the GUI may be displayed via a microscope integrated display (MID). In another example, the GUI may be displayed via a display. A surgeon may view and/or interact with the GUI via the MID. The surgeon and/or other surgical personnel may interact with the GUI via the display. The GUI may be overlaid to the surgeon's current area of interest. For example, the GUI may overlay the surgeon's current area of interest so the surgeon may visualize the GUI without looking away from surgeon's current area of interest. For example, the GUI may overlay a live scene. Motion-based object tracking may be utilized in interacting with the GUI. For example, utilizing motion-based object tracking and/or object recognition, surgical tooling equipment may be utilized as a pointing device in interacting with the GUI. Examples of a pointing device may be or include one or more of a mouse, a trackpad, and a trackball, among others.

Surgical tooling equipment may be registered with a system to be utilized in association with the GUI. For example, surgical tooling equipment may be registered with the system to be utilized as of a pointing device to be utilized in association with the GUI. Registering the surgical tooling equipment to be utilized in association with the GUI may include the system receiving one or more images of the surgical tooling equipment and determining one or more shapes and/or one or more curves of the surgical tooling equipment that may be utilized in identifying the surgical tooling equipment. For example, one or more machine learning processes and/or one or more machine learning methods may be utilized in determining one or more shapes and/or one or more curves of the surgical tooling equipment that may be utilized in identifying the surgical tooling equipment. The one or more machine learning processes and/or one or more machine learning methods may produce and/or determine a digital model of the surgical tooling equipment. For example, the digital model may be utilized in inferring one or more positions of the surgical tooling equipment in associated utilization with the GUI.

One or more movements of the surgical tooling equipment may be utilized in determining a pointer "click". For example, one or more movements of the surgical tooling equipment may be utilized as a mouse click. The pointer "click" may indicate a selection of one or more items displayed via the GUI. After the surgical tooling equipment is registered with the system, One or more movements of the surgical tooling equipment may be determined and/or identified as a pointer "click". In one example, a first movement may be utilized as a left mouse button selection (e.g. "click"). In a second example, a second movement may be utilized as a right mouse button selection (e.g. "click"). In a third example, a third movement may be utilized as a left mouse button hold selection (e.g. holding down a left mouse button). In another example, a fourth movement may be utilized as a left mouse button release selection (e.g. releasing a left mouse button). One or more motion-based object tracking processes and/or one or more motion-based object tracking methods may be utilized. For example, the one or more motion-based object tracking processes and/or one or more motion-based object tracking methods may utilize one or more of background subtraction, frame difference, and optical flow, among others, to track surgical tooling equipment.

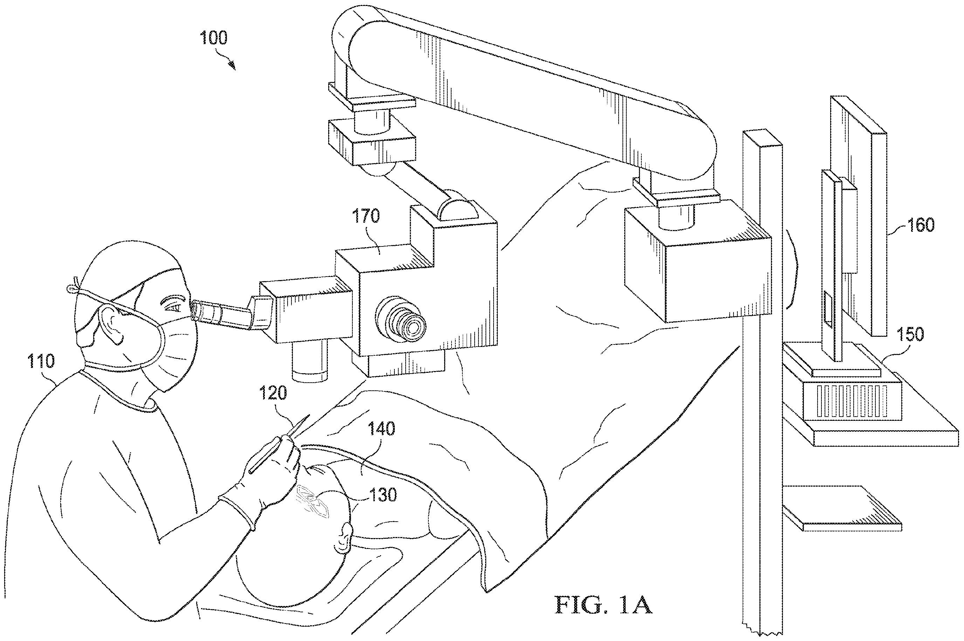

Turning now to FIG. 1A, an example of a system is illustrated. As shown, a surgeon 110 may utilize surgical tooling equipment 120. In one example, surgeon 110 may utilize surgical tooling equipment 120 in a surgery involving a patient portion 130 of a patient 140. For example, surgeon 110 may utilize surgical tooling equipment 120 in interacting with and/or utilizing a system 100. For example, system 100 may be or include an ophthalmic surgical tool tracking system. As illustrated, system 100 may include a computing device 150, a display 160, and a MID 170.

Computing device 150 may receive image frames captured by one or more image sensors. For example, computing device 150 may perform various image processing on the one or more image frames. Computing device 150 may perform image analysis on the one or more image frames to identify and/or extract one or more images of surgical tooling equipment 120 from the one or more image frames. Computing device 150 may generate a GUI, which may overlay the one or more image frames. For example, the GUI may include one or more indicators and/or one or more icons, among others. The one or more indicators may include surgical data, such as one or more positions and/or one or more orientations. The GUI may be displayed by display 160 and/or MID 170 to surgeon 110 and/or other medical personnel.

Computing device 150, display 160, and MID 170 may be implemented in separate housings communicatively coupled to one another or within a common console or housing. A user interface may be associated with one or more of computing device 150, display 160, and MID 170, among others. For example, a user interface may include one or more of a keyboard, a mouse, a joystick, a touchscreen, an eye tracking device, a speech recognition device, a gesture control module, dials, and/or buttons, among other input devices. A user (e.g., surgeon 110 and/or other medical personnel) may enter desired instructions and/or parameters via the user interface. For example, the user interface may be utilized in controlling one or more of computing device 150, display 160, and MID 170, among others.

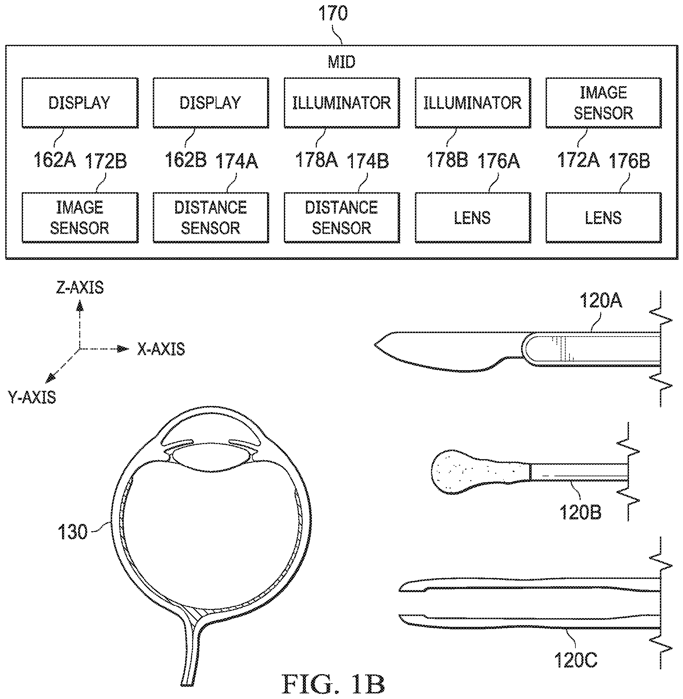

Turning now to FIG. 1B, an example of a microscope integrated display and examples of surgical tooling equipment are illustrated. As shown, surgical tooling equipment 120A may be or include a scalpel. As illustrated, surgical tooling equipment 120B may be or include a Q-tip. As shown, surgical tooling equipment 120C may be or include tweezers. Other surgical tooling equipment that is not specifically illustrated may be utilized with one or more systems, one or more processes, and/or one or more methods described herein.

As an example, surgical tooling equipment 120 may be marked with one or more patterns. The one or more patterns may be utilized in identifying surgical tooling equipment 120. The one or more patterns may include one or more of a hash pattern, a stripe pattern, and a fractal pattern, among others. As another example, surgical tooling equipment 120 may be marked with a dye and/or a paint. The dye and/or the paint may reflect one or more of visible light, infrared light, and ultraviolet light, among others. In one example, an illuminator 178 may provide ultraviolet light, and image sensor 172 may receive the ultraviolet light reflected from surgical tooling equipment 120. Computer system 150 may receive image data, based at least on the ultraviolet light reflected from surgical tooling equipment 120, from image sensor 172 and may utilize the image data, based at least on the ultraviolet light reflected from surgical tooling equipment 120, to identify surgical tooling equipment 120 from other image data provided by image sensor 172. In another example, an illuminator 178 may provide infrared light, and image sensor 172 may receive the infrared light reflected from surgical tooling equipment 120. Computer system 150 may receive image data, based at least on the infrared light reflected from surgical tooling equipment 120, from image sensor 172 and may utilize the image data, based at least on the infrared light reflected from surgical tooling equipment 120, to identify surgical tooling equipment 120 from other image data provided by image sensor 172.

As illustrated, MID 170 may include displays 162A and 162B. For example, surgeon 110 may look into multiple eye pieces, and displays 162A and 162B may display information to surgeon 110. Although MID 170 is shown with multiple displays, MID 170 may include a single display 162. For example, MID 170 may be implemented with one or more displays 162. As shown, MID 170 may include image sensors 172A and 172B. In one example, image sensors 172A and 172B may acquire images. In a second example, image sensors 172A and 172B may include cameras. In another example, an image sensor 172 may acquire images via one or more of visible light, infrared light, and ultraviolet light, among others. One or more image sensors 172A and 172B may provide data of images to computing device 150. Although MID 170 is shown with multiple image sensors, MID 170 may include a single image sensor 172. For example, MID 170 may be implemented with one or more image sensors 172.

As illustrated, MID 170 may include distance sensors 174A and 174. For example, a distance sensor 174 may determine a distance to surgical tooling equipment 120. Distance sensor 174 may determine a distance associated with a Z-axis. Although MID 170 is shown with multiple image sensors, MID 170 may include a single distance sensor 174. In one example, MID 170 may be implemented with one or more distance sensors 174. In another example, MID 170 may be implemented with no distance sensor. As shown, MID 170 may include lenses 176A and 176B. Although MID 170 is shown with multiple lenses 176A and 176B, MID 170 may include a single lens 176. For example, MID 170 may be implemented with one or more lenses 176. As illustrated, MID 170 may include illuminators 178A and 178B. For example, an illuminator 178 may provide and/or produce one or more of visible light, infrared light, and ultraviolet light, among others. Although MID 170 is shown with multiple illuminators, MID 170 may include a single illuminator 178. For example, MID 170 may be implemented with one or more illuminators 178.

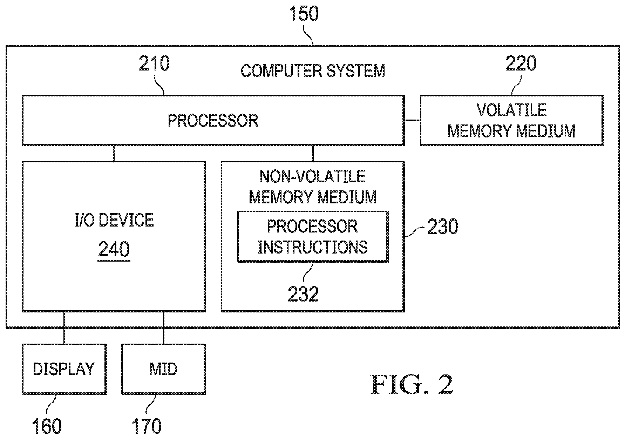

Turning now to FIG. 2, an example of a computer system is illustrated. As shown, computer system 150 may include a processor 210, a volatile memory medium 220, a non-volatile memory medium 230, and an input/output (I/O) device 240,. As illustrated, volatile memory medium 220, non-volatile memory medium 230, and I/O device 240 may be communicatively coupled to processor 210.

The term "memory medium" may mean a "memory", a "storage device", a "memory device", a "computer-readable medium", and/or a "tangible computer readable storage medium". For example, a memory medium may include, without limitation, storage media such as a direct access storage device, including a hard disk drive, a sequential access storage device, such as a tape disk drive, compact disk (CD), random access memory (RAM), read-only memory (ROM), CD-ROM, digital versatile disc (DVD), electrically erasable programmable read-only memory (EEPROM), flash memory, non-transitory media, and/or one or more combinations of the foregoing. As shown, non-volatile memory medium 230 may include processor instructions 232. Processor instructions 232 may be executed by processor. In one example, one or more portions of processor instructions 232 may be executed via non-volatile memory medium 230. In another example, one or more portions of processor instructions 232 may be executed via volatile memory medium 220. One or more portions of processor instructions 232 may be transferred to volatile memory medium 220.

Processor 210 may execute processor instructions 232 in implementing one or more systems, one or more flow charts, one or more processes, and/or one or more methods described herein. For example, processor instructions 232 may be configured, coded, and/or encoded with instructions in accordance with one or more systems, one or more flowcharts, one or more methods, and/or one or more processes described herein. One or more of a storage medium and a memory medium may be a software product, a program product, and/or an article of manufacture. For example, the software product, the program product, and/or the article of manufacture may be configured, coded, and/or encoded with instructions, executable by a processor, in accordance with one or more flowcharts, one or more methods, and/or one or more processes described herein.

Processor 210 may include any suitable system, device, or apparatus operable to interpret and execute program instructions, process data, or both stored in a memory medium and/or received via a network. Processor 210 further may include one or more microprocessors, microcontrollers, digital signal processors (DSPs), application specific integrated circuits (ASICs), or other circuitry configured to interpret and execute program instructions, process data, or both.

I/O device 240 may include any instrumentality or instrumentalities, which allow, permit, and/or enable a user to interact with computer system 150 and its associated components by facilitating input from a user and output to a user. Facilitating input from a user may allow the user to manipulate and/or control computer system 150, and facilitating output to a user may allow computer system 150 to indicate effects of the user's manipulation and/or control. For example, I/O device 240 may allow a user to input data, instructions, or both into computer system 150, and otherwise manipulate and/or control computer system 150 and its associated components. I/O devices may include user interface devices, such as a keyboard, a mouse, a touch screen, a joystick, a handheld lens, a tool tracking device, a coordinate input device, or any other I/O device suitable to be used with a system, such as system 100.

I/O device 240 may include one or more busses, one or more serial devices, and/or one or more network interfaces, among others, that may facilitate and/or permit processor 210 to implement one or more systems, processes, and/or methods described herein. In one example, I/O device 240 may include a storage interface that may facilitate and/or permit processor 210 to communicate with an external storage. The storage interface may include one or more of a universal serial bus (USB) interface, a SATA (Serial ATA) interface, a PATA (Parallel ATA) interface, and a small computer system interface (SCSI), among others. In a second example, I/O device 240 may include a network interface that may facilitate and/or permit processor 210 to communicate with a network. I/O device 240 may include one or more of a wireless network interface and a wired network interface. In a third example, I/O device 240 may include one or more of a peripheral component interconnect (PCI) interface, a PCI Express (PCIe) interface, a serial peripheral interconnect (SPI) interface, and an inter-integrated circuit (I²C) interface, among others. In another example, I/O device 240 may facilitate and/or permit processor 210 to communicate data with one or more of display 160 and MID 170, among others.

As shown, I/O device 240 may be communicatively coupled to display 160 and MID 170. For example, computer system 150 may be communicatively coupled to display 160 and MID 170 via I/O device 240. I/O device 240 may facilitate and/or permit processor 210 to communicate data with one or more elements of MID 170. In one example, I/O device 240 may facilitate and/or permit processor 210 to communicate data with one or more of an image sensor 172, a distance sensor 174, and a display 162, among others. In another example, I/O device 240 may facilitate and/or permit processor 210 to control one or more of an image sensors 172, a distance sensor 174, an illuminator 178, and a display 162, among others.

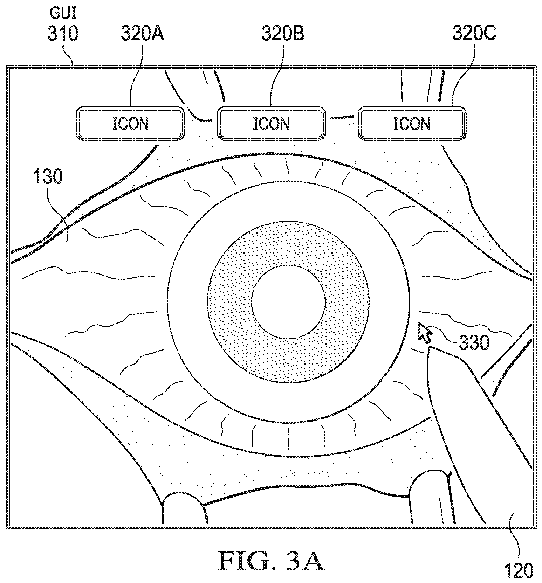

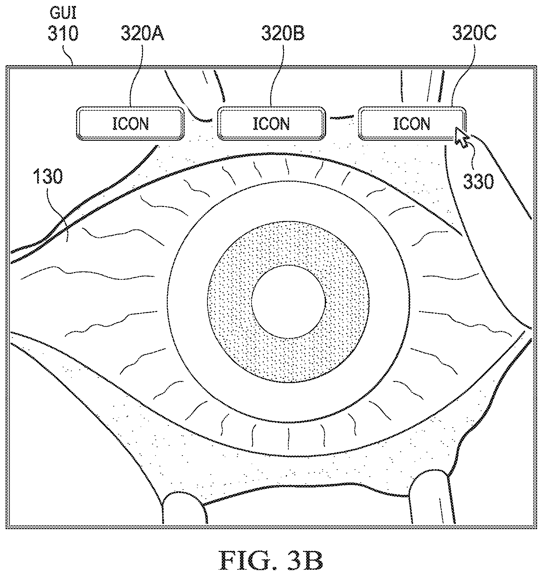

Turning now to FIGs. 3A and 3B, examples of a graphical user interface are illustrated. As shown, a GUI 310 may include icons 320A-320C. For example, GUI 310 and/or icons 320A-320C may be overlaid on an image acquired via an image sensor 172. As illustrated, GUI 310 may display a cursor 330. For example, system 100 may track movements of surgical tooling equipment 120 and display cursor 330 based one or more movements and/or one or more positions of surgical tooling equipment 120. System 100 may track movements of surgical tooling equipment 120. For example, system 100 may track one or more movements and/or one or more positions of surgical tooling equipment 120 to icon 320C.

GUI 310 may be displayed via a display. For example, GUI 310 may be displayed via one or more of displays 160, 162A, and 162B, among others. Surgeon 110 may select icon 320C. In one example, surgeon 110 may select icon 320C via a foot pedal. An actuation of a foot pedal may be utilized as a pointer click (e.g., a mouse click). In another example, surgeon 110 may select icon 320C via one or more movements of surgical tooling equipment 120. The one or more movements of surgical tooling equipment 120 may be utilized as a pointer click (e.g., a mouse click).

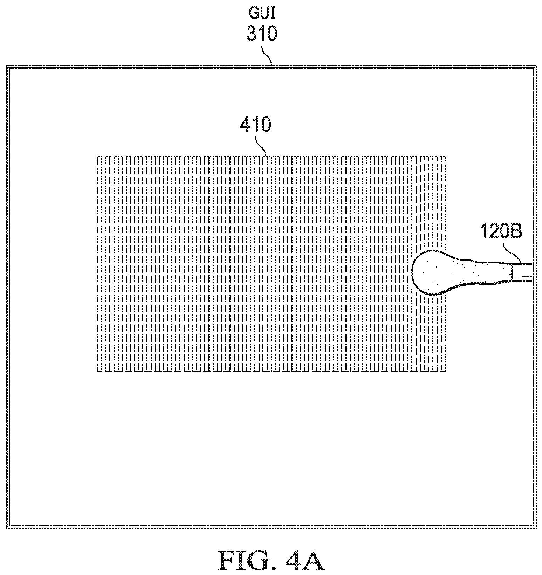

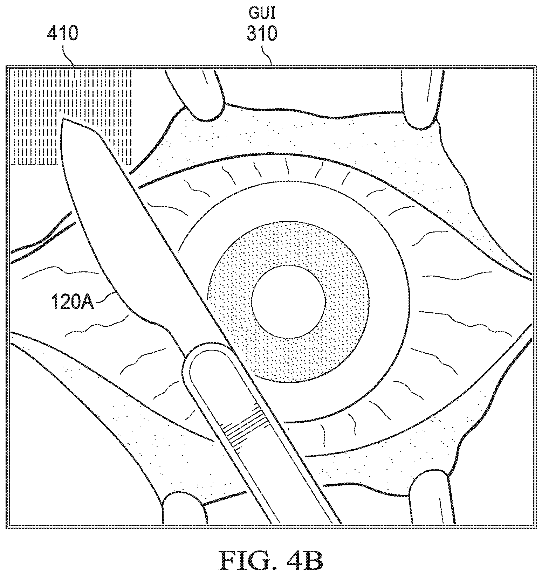

Turning now to FIGs. 4A and 4B, examples of registration areas are illustrated. As shown in FIG. 4A, surgical tooling equipment 120B may be registered via a registration area 410. For example, registration area 410 may be displayed via GUI 310. As illustrated in FIG. 4B, surgical tooling equipment 120A may be registered via registration area 410. For example, via registration area 410 may overlay an acquired image. The acquired image may have been acquired via an image sensor 172A and 172B.

A digital model of surgical tooling equipment 120 may be determined from one or more images from one or more image sensors 172. The digital model of surgical tooling equipment 120 may include a pattern of surgical tooling equipment 120. As an example, the digital model may be utilized in relating image data of surgical tooling equipment 120 within an image acquired via one or more of image sensors 172A and 172B. The digital model may include possible relationships between image data of the surgical tooling equipment within an image acquired via one or more of image sensors 172A and 172B. For example, digital model may include parameters may determine the possible relationships. A learning process and/or method may fit the parameters utilizing training data. In one example, one or more images may be utilized as training data. In another example, registration area 410 may be utilized in associating image data as training data. Determining the digital model of surgical tooling equipment 120 may include training the digital model based at least on the one or more images. The digital model may be discriminative. The digital model may be generative. One or more inference processes and/or one or more methods may utilize the digital model to determine image data of surgical tooling equipment 120 within an image acquired via one or more of image sensors 172A and 172B.

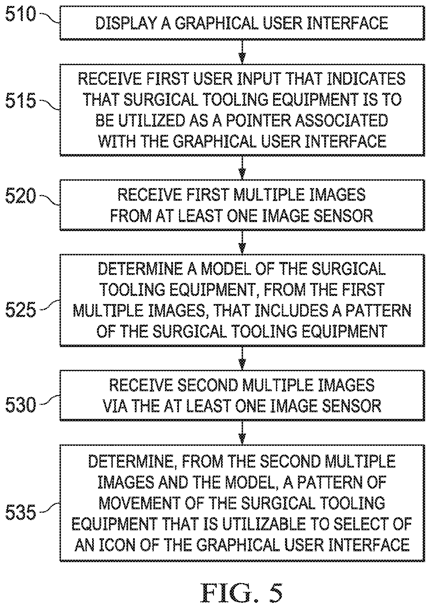

Turning now to FIG. 5, an example of a method utilizing surgical tooling equipment with a graphical user interface is illustrated. At 510, a graphical user interface may be displayed via a display. In one example, GUI 310 may be displayed via display 160. In another example, GUI 310 may be displayed via one or more of display 162A and 162B. At 515, first user input that indicates that surgical tooling equipment is to be utilized as a pointer associated with the graphical user interface may be received. In one example, the first user input may include an actuation of a foot pedal. In a second example, the first user input may include voice input. In another example, the first user input may include an actuation of a GUI icon. Surgeon 110 or other medical personnel may actuate the GUI icon.

At 520, first multiple images from at least one image sensor may be received. For example, first multiple images from one or more of image sensors 172A and 172B may be received. The first multiple images may include image data of the surgical tooling equipment that is to be utilized as the pointer associated with the graphical user interface. At 525, a digital model, that includes a pattern of the surgical tooling equipment, of the surgical tooling equipment, may be determined from the first multiple images. For example, the digital model may be utilized in relating image data of the surgical tooling equipment within an image acquired via one or more of image sensors 172A and 172B. The digital model may include possible relationships between image data of the surgical tooling equipment within an image acquired via one or more of image sensors 172A and 172B. For example, digital model may include parameters may determine the possible relationships. A learning process and/or method may fit the parameters utilizing training data. For example, the first multiple images may be utilized as training data. Determining the digital model of surgical tooling equipment may include training the digital model based at least on the first multiple images. The digital model may be discriminative. The digital model may be generative. An inference process and/or method may utilize the digital model to determine image data of the surgical tooling equipment within an image acquired via one or more of image sensors 172A and 172B.

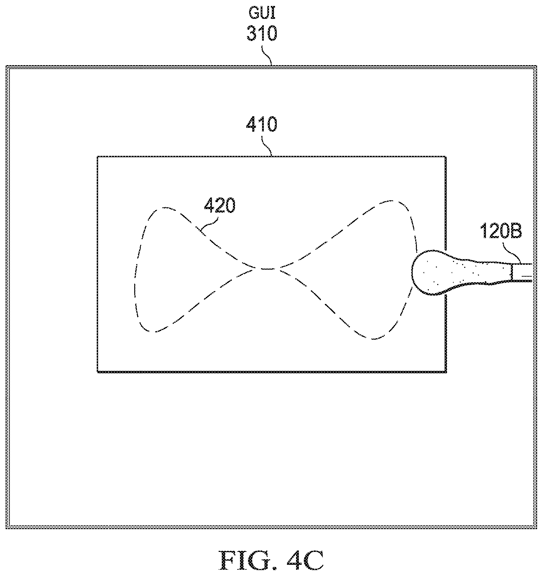

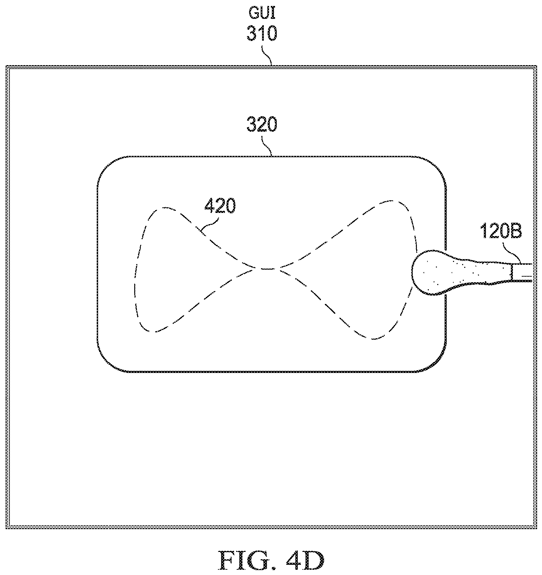

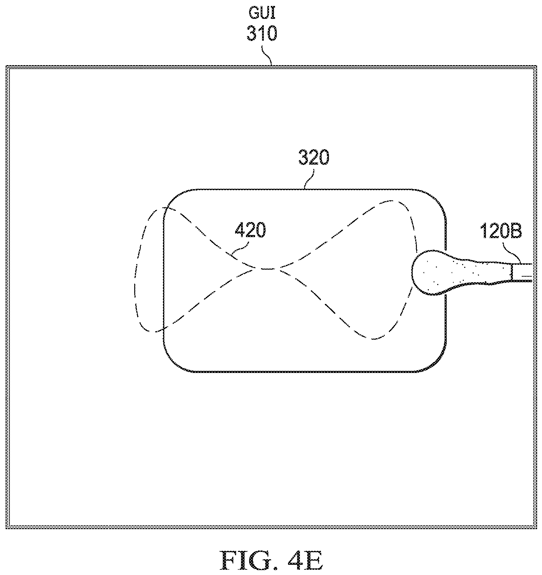

At 530, second multiple images may be received via the at least one image sensor. For example, second multiple images from one or more of image sensors 172A and 172B may be received. The second multiple images may include image data of the surgical tooling equipment. At 535, a pattern of movement of the surgical tooling equipment that is utilizable to select of an icon of the graphical user interface may be determined from the second multiple images and the digital model. For example, a pattern 420, illustrated in FIG. 4C, may be determined from the second multiple images and the digital model. Pattern 420 may be utilized to select an icon 320, as shown in FIG. 4D. Pattern 420 may be utilized to select an icon 320, as shown in FIG. 4E. For example, at least a portion of pattern 420 may overlap icon 420.

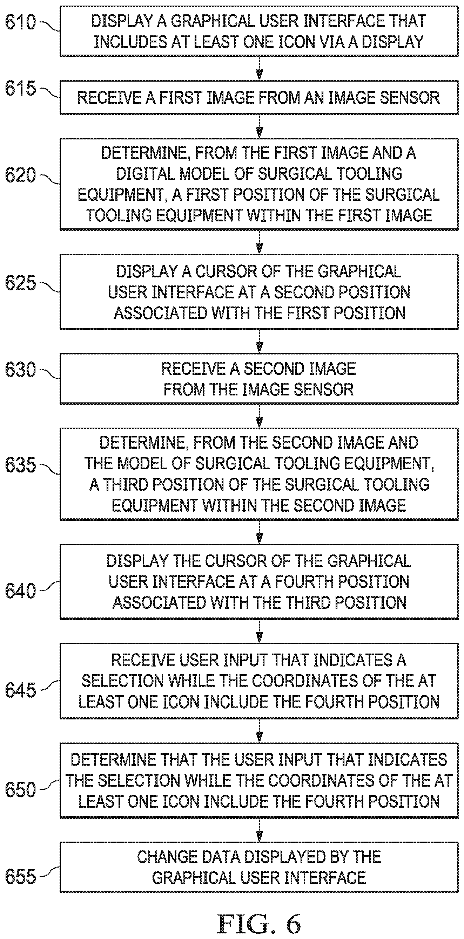

Turning now to FIG. 6, another example of a method utilizing surgical tooling equipment with a graphical user interface is illustrated. At 610, a graphical user interface that includes at least one icon may be displayed via a display. In one example, GUI 310 may be displayed via display 160. In another example, GUI 310 may be displayed via one or more of display 162A and 162B. At 615, a first image from an image sensor may be received. For example, a first image from image sensor 172 may be received. At 620, a first position of the surgical tooling equipment within the first image may be determined from the first image and a digital model of surgical tooling equipment. For example, first position of surgical tooling equipment 120, shown in FIG. 3A, may be determined from the first image and a digital model of surgical tooling equipment. As an example, the digital model may be or include the digital model determined via method element 525 of FIG. 5. As another example, the digital model may be retrieved from a memory medium. A memory medium may store one or more digital models of surgical tooling equipment. For example, the memory medium may store a library that includes one or more digital models of surgical tooling equipment.

At 625, a cursor of the graphical user interface at a second position associated with the first position may be displayed. For example, cursor 330 of GUI 310, shown in FIG. 3A, may be displayed at a second position associated with the first position. At 630, a second image from the image sensor may be received. For example, a second image from image sensor 172 may be received. At 635, a third position of the surgical tooling equipment within the second image may be determined from the second image and the digital model of surgical tooling equipment. For example, a second position of surgical tooling equipment 120, shown in FIG. 3B, may be determined from the second image and the digital model of surgical tooling equipment. At 640, the cursor of the graphical user interface may be displayed at a fourth position associated with the third position. For example, cursor 330 of GUI 310, shown in FIG. 3B, may be displayed at a fourth position associated with the third position.

At 645, user input that indicates a selection, while coordinates of the at least one icon include the fourth position, may be received. In one example, the user input may include an actuation of a foot pedal. Surgeon 110 may actuate the foot pedal as the user input that indicates the selection. In a second example, the user input may include a movement pattern. The user input may include movement pattern 420 shown in FIGs. 4D and 4E. The movement pattern may be approximate to movement pattern 420 shown in FIGs. 4D and 4E. Other movement patterns may be configured and/or utilized. In a third example, the user input may include a change in a number of pixels associated with the surgical tooling equipment that indicates a change in distance of the surgical tooling equipment to the image sensor. A number of pixels associated with the surgical tooling equipment may increase if the surgical tooling equipment is brought closer to the image sensor. As an example, receive a third image from the image sensor from the image sensor may be received, and a change in a number of pixels associated with the surgical tooling equipment that indicates a change in distance of the surgical tooling equipment to the image sensor may be determined based at least on the second and third images and the digital model.

At 650, it may be determined that the user input that indicates the selection while the coordinates of the at least one icon include the fourth position. For example, it may be determined that the user input that indicates a selection of icon 320. At 655, data displayed by the graphical user interface may be changed. For example, image data of GUI 310 may be changed. Changing the data displayed by the graphical user interface may be performed in response to determining that the user input that indicates the selection. As an example, a workflow associated with a surgery may proceed to a next step of the workflow. Image data of GUI 310 may be changed in association with the next step of the workflow associated with the surgery. As another example, at least a portion of the first image, the second image, or the third image may be stored in response to determining that the user input that indicates the selection. Image data may be stored via a memory medium.

One or more of the method and/or process elements and/or one or more portions of a method and/or processor elements may be performed in varying orders, may be repeated, or may be omitted. Furthermore, additional, supplementary, and/or duplicated method and/or process elements may be implemented, instantiated, and/or performed as desired. Moreover, one or more of system elements may be omitted and/or additional system elements may be added as desired.

A memory medium may be and/or may include an article of manufacture. For example, the article of manufacture may include and/or may be a software product and/or a program product. The memory medium may be coded and/or encoded with processor-executable instructions in accordance with one or more flowcharts, systems, methods, and/or processes described herein to produce the article of manufacture.

## Claims

1. A system (100) for allowing use of a surgical tooling equipment as a pointer associated with a graphical user interface, the system (100) comprising:
at least one processor (210); and
a memory medium (230) that is coupled to the at least one processor (210) and that includes instructions, when executed by the at least one processor (210), cause the system (100) to:
display the graphical user interface (310) via a display (160, 162A-B);
display an overlay on the graphical user interface (310), the overlay comprising a registration area (410) for the surgical tooling equipment (120), wherein the registration area (410) is used for receiving one or more images of the surgical tooling equipment (120) and determining one or more shapes and/or one or more curves of the surgical tooling equipment (120) that may be utilized in identifying the surgical tooling equipment (120);
receive first user input that indicates that the surgical tooling equipment (120) is to be utilized as the pointer associated with the graphical user interface (310);
receive a first plurality of images from at least one image sensor (172A-B);
determine a digital model of the surgical tooling equipment (120), from the first plurality of images and the one or more images of the surgical tooling equipment (120) from the registration area (410), that includes a pattern of the surgical tooling equipment (120);
train the digital model based at least on the first plurality of images; and
use the trained digital model to infer one or more positions of the surgical tooling equipment (120) in associated utilization with the graphical user interface (310).

2. The system (100) of claim 1, wherein, to train the digital model based at least on the first plurality of images, the instructions further cause the system (100) to determine data associated with the surgical tooling equipment (120) in a portion of each of the first plurality of images.

3. The system (100) of claim 2, wherein, to display the graphical user interface (310) via the display (160, 162A-B), the instructions further cause the system (100) to indicate an area, via the graphical user interface (310), associated with each portion of each of the first plurality of images.

4. The system (100) of claim 1, wherein the instructions further cause the system (100) to:
receive a second plurality of images via the at least one image sensor (172A-B); and
determine, from the second plurality of images and the digital model, a pattern of movement of the surgical tooling equipment (120) that is utilizable to select of an icon (320A-C) of the graphical user interface (310).

5. At least one non-transitory computer readable storage medium that includes instructions that, when executed by a processor (210) of a system (100), the system (100) for allowing use of a surgical tooling equipment as a pointer associated with a graphical user interface, cause the system (100) to:
display the graphical user interface (310) via a display (160, 162A-B);
display an overlay on the graphical user interface (310), the overlay comprising a registration area (410) for the surgical tooling equipment (120), wherein the registration area (410) is used for receiving one or more images of the surgical tooling equipment (120) and determining one or more shapes and/or one or more curves of the surgical tooling equipment (120) that may be utilized in identifying the surgical tooling equipment (120);
receive first user input that indicates that the surgical tooling equipment (120) is to be utilized as the pointer associated with the graphical user interface (310);
receive a first plurality of images from at least one image sensor (172A-B);
determine a digital model of the surgical tooling equipment (120), from the first plurality of images, and the one or more images of the surgical tooling equipment (120) from the registration area (410) that includes a pattern of the surgical tooling equipment (120);
train the digital model based at least on the first plurality of images; and
use the trained digital model to infer one or more positions of the surgical tooling equipment (120) in associated utilization with the graphical user interface (310).

6. The at least one non-transitory computer readable storage medium of claim 5, wherein, to train the digital model based at least on the first plurality of images, the instructions further cause the system (100) to determine data associated with the surgical tooling equipment (120) in a portion of each of the first plurality of images.

7. The at least one non-transitory computer readable storage medium of claim 6, wherein, to display the graphical user interface (310) via the display (160, 162A-B), the instructions further cause the system (100) to indicate an area, via the graphical user interface (310), associated with each portion of each of the first plurality of images.

8. The at least one non-transitory computer readable storage medium of claim 5, wherein the instructions further cause the system (100) to:
receive a second plurality of images via the at least one image sensor (172A-B); and
determine, from the second plurality of images and the digital model, a pattern of movement of the surgical tooling equipment (120) that is utilizable to select of an icon of the graphical user interface (310).

9. A method for allowing use of a surgical tooling equipment as a pointer associated with a graphical user interface, the method comprising:
displaying the graphical user interface (310) via a display (160, 162A-B);
displaying an overlay on the graphical user interface (310), the overlay comprising a registration area (410) for the surgical tooling equipment (120), wherein the registration area (410) is used for receiving one or more images of the surgical tooling equipment (120) and determining one or more shapes and/or one or more curves of the surgical tooling equipment (120) that may be utilized in identifying the surgical tooling equipment (120);
receiving first user input that indicates that surgical tooling equipment (120) is to be utilized as the pointer associated with the graphical user interface (310);
receiving a first plurality of images from at least one image sensor (172A-B);
determining a digital model of the surgical tooling equipment (120), from the first plurality of images, and the one or more images of the surgical tooling equipment (120) from the registration area (410) that includes a pattern of the surgical tooling equipment (120);
training the digital model based at least on the first plurality of images; and
using the trained digital model to infer one or more positions of the surgical tooling equipment (120) in associated utilization with the graphical user interface (310).

10. The method of claim 9, wherein the training the digital model based at least on the first plurality of images includes determining data associated with the surgical tooling equipment (120) in a portion of each of the first plurality of images.

11. The method of claim 10, wherein the displaying the graphical user interface (310) via the display (160, 162A-B) includes indicating an area, via the graphical user interface (310), associated with each portion of each of the first plurality of images.

12. The method of claim 9, further comprising:
receiving a second plurality of images via the at least one image sensor (172A-B); and
determining, from the second plurality of images and the digital model, a pattern of movement of the surgical tooling equipment (120) that is utilizable to select of an icon of the graphical user interface (310).

## Patentansprüche

1. System (100) zum Ermöglichen der Verwendung eines chirurgischen Werkzeugs als Zeiger im Zusammenhang mit einer grafischen Benutzeroberfläche, wobei das System (100) Folgendes umfasst:
mindestens einen Prozessor (210); und
ein Speichermedium (230), das mit dem mindestens einen Prozessor (210) gekoppelt ist und das Anweisungen beinhaltet, die bei Ausführung durch den mindestens einen Prozessor (210) bewirken, dass das System (100) Folgendes durchführt:
Anzeigen der grafischen Benutzeroberfläche (310) über eine Anzeige (160, 162A-B);
Anzeigen einer Überlagerung auf der grafischen Benutzeroberfläche (310), wobei die Überlagerung einen Registrierungsbereich (410) für das chirurgische Werkzeug (120) umfasst, wobei der Registrierungsbereich (410) dazu verwendet wird, ein oder mehrere Bilder des chirurgischen Werkzeugs (120) zu empfangen und eine oder mehrere Formen und/oder eine oder mehrere Krümmungen des chirurgischen Werkzeugs (120) zu bestimmen, die bei der Identifizierung des chirurgischen Werkzeugs (120) verwendet werden können;
Empfangen einer ersten Benutzereingabe, die angibt, dass das chirurgische Werkzeug (120) als der Zeiger im Zusammenhang mit der grafischen Benutzeroberfläche (310) verwendet werden soll;
Empfangen einer ersten Vielzahl von Bildern von mindestens einem Bildsensor (172A-B);
Bestimmen eines digitalen Modells des chirurgischen Werkzeugs (120) anhand der ersten Vielzahl von Bildern und des einen oder der mehreren Bilder des chirurgischen Werkzeugs (120) aus dem Registrierungsbereich (410), das ein Muster des chirurgischen Werkzeugs (120) beinhaltet;
Trainieren des digitalen Modells basierend mindestens auf der ersten Vielzahl von Bildern; und
Verwenden des trainierten digitalen Modells zum Ableiten einer oder mehrerer Positionen des chirurgischen Werkzeugs (120) bei assoziierter Verwendung mit der grafischen Benutzeroberfläche (310).

2. System (100) nach Anspruch 1, wobei die Anweisungen, um das digitale Modell basierend mindestens auf der ersten Vielzahl von Bildern zu trainieren, ferner bewirken, dass das System (100) Daten im Zusammenhang mit dem chirurgischen Werkzeug (120) in einem Teil jedes der ersten Vielzahl von Bildern bestimmt.

3. System (100) nach Anspruch 2, wobei die Anweisungen, um die grafische Benutzeroberfläche (310) über die Anzeige (160, 162A-B) anzuzeigen, ferner bewirken, dass das System (100) über die grafische Benutzeroberfläche (310) einen Bereich im Zusammenhang mit jedem Teil jedes der ersten Vielzahl von Bildern angibt.

4. System (100) nach Anspruch 1, wobei die Anweisungen ferner bewirken, dass das System (100) Folgendes durchführt:
Empfangen einer zweiten Vielzahl von Bildern über den mindestens einen Bildsensor (172A-B); und
Bestimmen, anhand der zweiten Vielzahl von Bildern und des digitalen Modells, eines Bewegungsmusters des chirurgischen Werkzeugs (120), das dazu verwendet werden kann, ein Symbol (320A-C) der grafischen Benutzeroberfläche (310) auszuwählen.

5. Mindestens ein nichtflüchtiges computerlesbares Speichermedium, das Anweisungen umfasst, die bei Ausführung durch einen Prozessor (210) eines Systems (100), wobei das System (100) zum Ermöglichen der Verwendung eines chirurgischen Werkzeugs als Zeiger im Zusammenhang mit einer grafischen Benutzeroberfläche dient, bewirken, dass das System (100) Folgendes durchführt:
Anzeigen der grafischen Benutzeroberfläche (310) über eine Anzeige (160, 162A-B);
Anzeigen einer Überlagerung auf der grafischen Benutzeroberfläche (310), wobei die Überlagerung einen Registrierungsbereich (410) für das chirurgische Werkzeug (120) umfasst, wobei der Registrierungsbereich (410) dazu verwendet wird, ein oder mehrere Bilder des chirurgischen Werkzeugs (120) zu empfangen und eine oder mehrere Formen und/oder eine oder mehrere Krümmungen des chirurgischen Werkzeugs (120) zu bestimmen, die bei der Identifizierung des chirurgischen Werkzeugs (120) verwendet werden können;
Empfangen einer ersten Benutzereingabe, die angibt, dass das chirurgische Werkzeug (120) als der Zeiger im Zusammenhang mit der grafischen Benutzeroberfläche (310) verwendet werden soll;
Empfangen einer ersten Vielzahl von Bildern von mindestens einem Bildsensor (172A-B);
Bestimmen eines digitalen Modells des chirurgischen Werkzeugs (120) anhand der ersten Vielzahl von Bildern und des einen oder der mehreren Bilder des chirurgischen Werkzeugs (120) aus dem Registrierungsbereich (410), das ein Muster des chirurgischen Werkzeugs (120) beinhaltet;
Trainieren des digitalen Modells basierend mindestens auf der ersten Vielzahl von Bildern; und
Verwenden des trainierten digitalen Modells zum Ableiten einer oder mehrerer Positionen des chirurgischen Werkzeugs (120) bei assoziierter Verwendung mit der grafischen Benutzeroberfläche (310).

6. Mindestens ein nichtflüchtiges computerlesbares Speichermedium nach Anspruch 5, wobei die Anweisungen, um das digitale Modell basierend mindestens auf der ersten Vielzahl von Bildern zu trainieren, ferner bewirken, dass das System (100) Daten im Zusammenhang mit dem chirurgischen Werkzeug (120) in einem Teil jedes der ersten Vielzahl von Bildern bestimmt.

7. Mindestens ein nichtflüchtiges computerlesbares Speichermedium nach Anspruch 6, wobei die Anweisungen, um die grafische Benutzeroberfläche (310) über die Anzeige (160, 162A-B) anzuzeigen, ferner bewirken, dass das System (100) über die grafische Benutzeroberfläche (310) einen Bereich im Zusammenhang mit jedem Teil jedes der ersten Vielzahl von Bildern angibt.

8. Mindestens ein nichtflüchtiges computerlesbares Speichermedium nach Anspruch 5, wobei die Anweisungen ferner bewirken, dass das System (100) Folgendes durchführt:
Empfangen einer zweiten Vielzahl von Bildern über den mindestens einen Bildsensor (172A-B); und
Bestimmen, anhand der zweiten Vielzahl von Bildern und des digitalen Modells, eines Bewegungsmusters des chirurgischen Werkzeugs (120), das dazu verwendet werden kann, ein Symbol der grafischen Benutzeroberfläche (310) auszuwählen.

9. Verfahren zum Ermöglichen der Verwendung eines chirurgischen Werkzeugs als Zeiger im Zusammenhang mit einer grafischen Benutzeroberfläche, wobei das Verfahren Folgendes umfasst:
Anzeigen der grafischen Benutzeroberfläche (310) über eine Anzeige (160, 162A-B);
Anzeigen einer Überlagerung auf der grafischen Benutzeroberfläche (310), wobei die Überlagerung einen Registrierungsbereich (410) für das chirurgische Werkzeug (120) umfasst, wobei der Registrierungsbereich (410) dazu verwendet wird, ein oder mehrere Bilder des chirurgischen Werkzeugs (120) zu empfangen und eine oder mehrere Formen und/oder eine oder mehrere Krümmungen des chirurgischen Werkzeugs (120) zu bestimmen, die bei der Identifizierung des chirurgischen Werkzeugs (120) verwendet werden können;
Empfangen einer ersten Benutzereingabe, die angibt, dass das chirurgische Werkzeug (120) als der Zeiger im Zusammenhang mit der grafischen Benutzeroberfläche (310) verwendet werden soll;
Empfangen einer ersten Vielzahl von Bildern von mindestens einem Bildsensor (172A-B);
Bestimmen eines digitalen Modells des chirurgischen Werkzeugs (120) anhand der ersten Vielzahl von Bildern und des einen oder der mehreren Bilder des chirurgischen Werkzeugs (120) aus dem Registrierungsbereich (410), das ein Muster des chirurgischen Werkzeugs (120) beinhaltet;
Trainieren des digitalen Modells basierend mindestens auf der ersten Vielzahl von Bildern; und
Verwenden des trainierten digitalen Modells zum Ableiten einer oder mehrerer Positionen des chirurgischen Werkzeugs (120) bei assoziierter Verwendung mit der grafischen Benutzeroberfläche (310).

10. Verfahren nach Anspruch 9, wobei das Trainieren des digitales Modells basierend mindestens auf der ersten Vielzahl von Bildern umfasst, Daten im Zusammenhang mit dem chirurgischen Werkzeug (120) in einem Teil jedes der ersten Vielzahl von Bildern zu bestimmen.

11. Verfahren nach Anspruch 10, wobei das Anzeigen der grafischen Benutzeroberfläche (310) über die Anzeige (160, 162A-B) umfasst, über die grafische Benutzeroberfläche (310) einen Bereich im Zusammenhang mit jedem Teil jedes der ersten Vielzahl von Bildern anzugeben.

12. Verfahren nach Anspruch 9, das ferner Folgendes umfasst:
Empfangen einer zweiten Vielzahl von Bildern über den mindestens einen Bildsensor (172A-B); und
Bestimmen, anhand der zweiten Vielzahl von Bildern und des digitalen Modells, eines Bewegungsmusters des chirurgischen Werkzeugs (120), das dazu verwendet werden kann, ein Symbol der grafischen Benutzeroberfläche (310) auszuwählen.

## Revendications

1. Système (100) permettant l'utilisation d'un équipement d'outillage chirurgical comme un pointeur associé à une interface utilisateur graphique, le système (100) comprenant :
au moins un processeur (210) ; et
un support de mémoire (230) couplé à l'au moins un processeur (210) et comprenant des instructions qui, lorsqu'elles sont exécutées par l'au moins un processeur (210), amènent le système (100) à :
afficher l'interface utilisateur graphique (310) par l'intermédiaire d'un dispositif d'affichage (160, 162A-B) ;
afficher une superposition sur l'interface utilisateur graphique (310), la superposition comprenant une zone d'enregistrement (410) pour l'équipement d'outillage chirurgical (120), la zone d'enregistrement (410) étant utilisée pour recevoir une ou plusieurs images de l'équipement d'outillage chirurgical (120) et déterminer une ou plusieurs formes et/ou une ou plusieurs courbes de l'équipement d'outillage chirurgical (120) qui peuvent être utilisées pour identifier l'équipement d'outillage chirurgical (120) ;
recevoir une première entrée utilisateur indiquant que l'équipement d'outillage chirurgical (120) doit être utilisé comme le pointeur associé à l'interface utilisateur graphique (310) ;
recevoir une première pluralité d'images provenant d'au moins un capteur d'images (172A-B) ;
déterminer un modèle numérique de l'équipement d'outillage chirurgical (120), à partir de la première pluralité d'images et de la ou des images de l'équipement d'outillage chirurgical (120) provenant de la zone d'enregistrement (410), qui comprend un motif de l'équipement d'outillage chirurgical (120) ;
entraîner le modèle numérique en se basant au moins sur la première pluralité d'images ; et
utiliser le modèle numérique entraîné pour déduire une ou plusieurs positions de l'équipement d'outillage chirurgical (120) dans l'utilisation associée à l'interface utilisateur graphique (310).

2. Système (100) selon la revendication 1, pour entraîner le modèle numérique basé au moins sur la première pluralité d'images, les instructions amenant en outre le système (100) à déterminer des données associées à l'équipement d'outillage chirurgical (120) dans une partie de chacune de la première pluralité d'images.

3. Système (100) selon la revendication 2, pour afficher l'interface utilisateur graphique (310) par l'intermédiaire du dispositif d'affichage (160, 162A-B), les instructions amenant en outre le système (100) à indiquer une zone, par l'intermédiaire de l'interface utilisateur graphique (310), associée à chaque partie de chacune de la première pluralité d'images.

4. Système (100) selon la revendication 1, les instructions amenant en outre le système (100) à :
recevoir une seconde pluralité d'images par l'intermédiaire de l'au moins un capteur d'images (172A-B) ; et
déterminer, à partir de la seconde pluralité d'images et du modèle numérique, un motif de mouvement de l'équipement d'outillage chirurgical (120) qui peut être utilisé pour sélectionner une icône (320A-C) de l'interface utilisateur graphique (310).

5. Au moins un support de stockage lisible par ordinateur non transitoire comprenant des instructions qui, lorsqu'elles sont exécutées par un processeur (210) d'un système (100), le système (100) permettant l'utilisation d'un équipement d'outillage chirurgical comme un pointeur associé à une interface utilisateur graphique, amènent le système (100) à :
afficher l'interface utilisateur graphique (310) par l'intermédiaire d'un dispositif d'affichage (160, 162A-B) ;
afficher une superposition sur l'interface utilisateur graphique (310), la superposition comprenant une zone d'enregistrement (410) pour l'équipement d'outillage chirurgical (120), la zone d'enregistrement (410) étant utilisée pour recevoir une ou plusieurs images de l'équipement d'outillage chirurgical (120) et déterminer une ou plusieurs formes et/ou une ou plusieurs courbes de l'équipement d'outillage chirurgical (120) qui peuvent être utilisées pour identifier l'équipement d'outillage chirurgical (120) ;
recevoir une première entrée utilisateur indiquant que l'équipement d'outillage chirurgical (120) doit être utilisé comme le pointeur associé à l'interface utilisateur graphique (310) ;
recevoir une première pluralité d'images provenant d'au moins un capteur d'images (172A-B) ;
déterminer un modèle numérique de l'équipement d'outillage chirurgical (120), à partir de la première pluralité d'images et de la ou des images de l'équipement d'outillage chirurgical (120) provenant de la zone d'enregistrement (410) qui comprend un motif de l'équipement d'outillage chirurgical (120) ;
entraîner le modèle numérique en se basant au moins sur la première pluralité d'images ; et
utiliser le modèle numérique formé pour déduire une ou plusieurs positions de l'équipement d'outillage chirurgical (120) dans l'utilisation associée à l'interface utilisateur graphique (310).

6. L'au moins un support de stockage lisible par ordinateur non transitoire selon la revendication 5, pour entraîner le modèle numérique basé au moins sur la première pluralité d'images, les instructions amenant en outre le système (100) à déterminer des données associées à l'équipement d'outillage chirurgical (120) dans une partie de chacune de la première pluralité d'images.

7. L'au moins un support de stockage lisible par ordinateur non transitoire selon la revendication 6, pour afficher l'interface utilisateur graphique (310) par l'intermédiaire du dispositif d'affichage (160, 162A-B), les instructions amenant en outre le système (100) à indiquer une zone, par l'intermédiaire de l'interface utilisateur graphique (310), associée à chaque partie de chacune de la première pluralité d'images.

8. L'au moins un support de stockage non transitoire lisible par ordinateur selon la revendication 5, les instructions amenant en outre le système (100) à :
recevoir une seconde pluralité d'images par l'intermédiaire d'au moins un capteur d'images (172A- B) ; et
déterminer, à partir de la seconde pluralité d'images et du modèle numérique, un motif de mouvement de l'équipement d'outillage chirurgical (120) qui peut être utilisé pour sélectionner une icône de l'interface utilisateur graphique (310).

9. Procédé pour permettre l'utilisation d'un équipement d'outillage chirurgical comme un pointeur associé à une interface utilisateur graphique, le procédé comprenant :
l'affichage de l'interface utilisateur graphique (310) par l'intermédiaire d'un dispositif d'affichage (160, 162A-B) ;
l'affichage d'une superposition sur l'interface utilisateur graphique (310), la superposition comprenant une zone d'enregistrement (410) pour l'équipement d'outillage chirurgical (120), la zone d'enregistrement (410) étant utilisée pour recevoir une ou plusieurs images de l'équipement d'outillage chirurgical (120) et déterminer une ou plusieurs formes et/ou une ou plusieurs courbes de l'équipement d'outillage chirurgical (120) qui peuvent être utilisées pour identifier l'équipement d'outillage chirurgical (120) ;
la réception d'une première entrée utilisateur indiquant que l'équipement d'outillage chirurgical (120) doit être utilisé comme le pointeur associé à l'interface utilisateur graphique (310) ;
la réception d'une première pluralité d'images provenant d'au moins un capteur d'images (172A-B) ;
la détermination d'un modèle numérique de l'équipement d'outillage chirurgical (120), à partir de la première pluralité d'images et de la ou des images de l'équipement d'outillage chirurgical (120) provenant de la zone d'enregistrement (410) qui comprend un motif de l'équipement d'outillage chirurgical (120) ;
l'entraînement du modèle numérique en se basant au moins sur la première pluralité d'images ; et
l'utilisation du modèle numérique entraîné pour déduire une ou plusieurs positions de l'équipement d'outillage chirurgical (120) dans l'utilisation associée à l'interface utilisateur graphique (310).

10. Procédé selon la revendication 9, l'entraînement du modèle numérique basé au moins sur la première pluralité d'images comprenant la détermination de données associées à l'équipement d'outillage chirurgical (120) dans une partie de chacune de la première pluralité d'images.

11. Procédé selon la revendication 10, l'affichage de l'interface utilisateur graphique (310) par l'intermédiaire du dispositif d'affichage (160, 162A-B) comprenant l'indication d'une zone, par l'intermédiaire de l'interface utilisateur graphique (310), associée à chaque portion de chacune de la première pluralité d'images.

12. Procédé selon la revendication 9, comprenant en outre :
la réception d'une seconde pluralité d'images par l'intermédiaire de l'au moins un capteur d'images (172A-B) ; et
la détermination, à partir de la seconde pluralité d'images et du modèle numérique, d'un motif de mouvement de l'équipement d'outillage chirurgical (120) qui peut être utilisé pour sélectionner une icône de l'interface utilisateur graphique (310).
